Europäisches Patentamt

European Patent Office

Office européen des brevets

(19)

(11) **EP 1 493 402 A2**

(12) # EUROPEAN PATENT APPLICATION

(43) Date of publication:
**05.01.2005 Bulletin 2005/01**

(51) Int Cl.7: **A61F 2/06**, B29D 23/00, D04C 1/06

(21) Application number: **04021681.4**

(22) Date of filing: **15.01.1998**

(84) Designated Contracting States:
**AT BE CH DE DK ES FR GB IE IT LI NL SE**

(30) Priority: **23.01.1997 US 36160 P**

(62) Document number(s) of the earlier application(s) in accordance with Art. 76 EPC:
**98300265.0 / 0 855 170**

(71) Applicant: **SCHNEIDER (USA) INC.
Plymouth, Minnesota 55442 (US)**

(72) Inventors:
• **Du, George
  Minnesota 55446 (US)**

• **Thompson, Paul
  Minnesota 55427 (US)**

(74) Representative: **Green, Mark Charles et al
Urquhart-Dykes & Lord LLP
30 Welbeck Street
London W1G 8ER (GB)**

Remarks:
This application was filed on 13 - 09 - 2004 as a divisional application to the application mentioned under INID code 62.

(54) **Stent graft with braided polymeric sleeve**

(57)    A stent graft (18) for transluminal implantation includes a resilient tubular interbraided latticework (34) metal or polymeric monofilaments, a tubular interbraided sleeve (40) of polymeric multifilament yarns, and an adhesive layer (44) between the sleeve (40) and latticework (34) for bonding them together including processes for fabricating the stent graft (18).

FIG-2

EP 1 493 402 A2

**Description**

BACKGROUND OF THE INVENTION

The present invention relates to body implantable devices and more particularly to prostheses incorporating the characteristics of stents and grafts and intended for long term intraluminal fixation.

A variety of patient treatment and diagnostic procedures involve devices intraluminally implanted into the body of a patient. Among these devices are stents as disclosed in U.S. Patent No. 4,655,771 (Wallsten). The devices in Wallsten are tubular, braided structures formed of helically wound thread elements. The stents are deployed using a delivery catheter such as discussed in U.S. Patent No. 5.026,377 (Burton et al.). With the stent positioned at the intended treatment site, an outer tube of the delivery catheter is withdrawn allowing the prosthesis to radially expand into a substantially conforming surface contact with a blood vessel wall or other tissue.

Thread elements or strands formed of metal are generally favored for applications requiring flexibility and effective resistance to radial compression after implantation. Metal strands can be thermally formed by a moderately high temperature age-hardening process while wound about a mandrel in the desired helical configuration. The strands, due to their high modulus of elasticity, cooperate to provide the needed strength. Strand flexibility also permits a radial compression and axial elongation of the stent that facilitates intraluminal delivery of the stent to the intended treatment site. Because the self-expanding device generally remains at least slightly radially compressed after fixation, its elastic restoring force can provide acute fixation.

The favorable combination of strength and flexibility is due largely to the properties of the strands. after they have been age-hardened, or otherwise thermally treated in the case of polymeric strands. The braiding angle of the helical strands and the axial spacing between adjacent strands also contribute to strength and flexibility. Age hardening processes are described in U.S. Patent No. 5,628,787 (Mayer) and U.S. Patent No. 5,645,559 (Hachtman et al.).

A well known alternative stent construction features plastically deformable metallic strands in lieu of resilient strands. Plastically deformable strands can be arranged in the same helical configuration. A plastically deformable stent requires no gripping members or other feature on the catheter to maintain the stent in a reduced-radius state during delivery. However, radial expansion of the stent at the treatment site requires a dilatation balloon or other expansion means.

Regardless of whether stents are self-expanding or plastically deformable, they characteristically have an open mesh construction, or otherwise are formed with multiple openings to facilitate the necessary radial enlargements and reductions and to allow tissue ingrowth of the metallic structure. Also, such stents characteristically longitudinally expand as they radially contract, and conversely radially expand as they longitudinally contract.

Devices featuring more tightly woven strands are known. For example, U.S. Patent No. 4,681,110 (Wiktor), discloses a flexible tubular liner insertable into the aorta to treat an aneurysm. The liner is a tight weave of flexible plastic strands, designed to elastically expand against the aneurysm to direct blood flow past the aneurysm. The tight weave is intended to minimize leakage, so that the liner can effectively shunt blood through to eliminate the aneurysmal sack from the blood path.

The Wiktor structure and others like it notwithstanding, those of skill in the art continue to encounter difficulty in providing a device that simultaneously accommodates the competing needs of low permeability, strength and flexibility for radial compression and expansion. One known approach to this problem is a combination stent graft, in which a compliant but substantially fixed-radius and tightly woven graft is sutured or otherwise coupled to a radially expandable stent. Upon release, the stent is intended to radially expand to the graft diameter. This requires a careful matching of the graft diameter with the lumen diameter at the treatment site. Otherwise, either an oversized graft is compressed between the stent and body tissue with undesirable folds or gathering of the graft material, or an undersized graft prevents the stent from radially expanding an amount sufficient to anchor the device.

Another difficulty arises from the fact that the stent layer and graft layer, even when both undergo combined radial contraction and axial elongation, behave according to different relationships governing the amount of radial reduction for a given axial increase. When the latticework elongates a greater amount for a given radial reduction, elongation of the composite structure tends to tear the bond joining the graft material to the stent. Conversely, if the graft layer undergoes the greater axial expansion, an unwanted increase in bending stiffness causes localized reductions in diameter when the stent graft is bent around tight radii. Consequently negotiation through tortuous vascular passageways becomes more difficult, and in some instances impossible.

The commercially available yarns used in textile vascular grafts are twisted for improved handling during weaving or knitting operations. The amount of twisting will depend upon certain factors including the process of yarn manufacture (e.g., continuous filament yarn or staple yarn) and desired denier. For continuous filament yarn processes, surface twisting angles will generally be between about 15-45 degrees. The multiple filaments typically form a substantially circular yarn cross-section. This limits the effectiveness of the stent graft, and increases the difficulty of matching the elongation behavior of the fabric graft, to that of the stent.

More particularly, the twisted multifilaments are tightly packed, yielding packing factors (or packing ratios) in the range of 0.7-0.9. Because of the tightly packed yarns, the tubular fabric graft has a tendency to kink when bent. The tightly packed filaments leave an insufficient void throughout the yarn cross-section for tissue ingrowth, reducing the effectiveness of long-term fixation. Further, the tightly packed yarn cross-section does not adjust itself in shape to accommodate axial elongation, thus limiting the radial contraction/axial elongation capability of the graft. The circular yarn cross-section further limits the elongation capability, because of its particular resistance to adjustments in shape.

Other disadvantages arise from the circular yarn cross-section. The yarn diameter determines the minimum thickness of the graft fabric. Yarn coverage typically is below 80 percent without additional compacting, and a fabric porosity usually is above 70 percent, again without additional compacting.

Several prostheses constructions had been suggested for composite braided structures that combine different types of strands, e.g. multifilament yarns, monofilaments, fusible materials and collagens. Examples are found in International Patent Publications No. WO 91/10766, No. WO 92/16166, No. WO 94/06372, and No. WO 94/06373. Further, a highly favorable combination of strength, resilience, range of treatable lumen diameters and low permeability has been achieved by two-dimensionally woven and three-dimensionally woven composite devices featuring textile strands interbraided with either selectively cold-worked or independently thermally set structural strands, as disclosed in U.S. Patent No. 5 758 562 and US Patent No 5 718 159, both filed April 30, 1996 and assigned to the assignee of this application. Although such devices are well suited for a wide range of procedures, there are costs and complexities inherent in interweaving different types of strands. Certain desirable modifications, e.g. providing selected areas of the device with only structural strands, are difficult.

US-A-5,575,815 (Pinchuk) discloses a braided tubular stent which may be coated or lined with a porous biocompatible material. The stent may be formed inside the tube. The coating can be bonded to the stent.

Therefore, it is an object of the present invention to provide a prosthesis structure that affords the advantages of stents and grafts, yet does not require an interbraiding of the structural strands characteristic of stents and the textile strands characteristic of grafts.

Another object is to provide a process for manufacturing a combination stent graft in which a structural layer and a low-permeability fabric layer undergo radial and axial enlargements and reductions, yet remain integrally bonded to one another.

It is a further object of the invention to provide a stent graft construction that facilitates selective alternate axial positioning of open-mesh areas and covered areas for shunting blood flow, to customize stent grafts for particular uses.

Yet another object is to provide, in a prosthesis featuring two or more layers formed of braided strands and of different materials, an effective bond to ensure that the layers remain integrally connected through radial expansions and contractions.

Another object is to provide a fabric graft that exhibits low permeability due to a high yarn coverage and low fabric porosity, in combination with a yarn cross-sectional porosity sufficient to enable tissue ingrowth.

A further object is to provide a stent graft with a fabric graft that is thinner and more closely matches the elongation behavior of the stent, yet affords acceptably low permeability.

SUMMARY OF THE INVENTION

To achieve the above and other objects, there is provided a process for making a stent graft, as defined in claim 1.

The step of applying an adhesive preferably involves a curable adhesive, applied uncured to the chosen one of the latticework and sleeve. Then, maintaining the latticework and sleeve in their engagement may further involve curing the adhesive to form the bond. A preferred manner of disposing one of the latticework and sleeve within the other is to adjust the selected one to reduce its radius below that in its nominal state, axially align it within the other while the other remains in its nominal state, then radially expand the selected one to achieve engagement of the latticework and sleeve.

Preferably the structural strands are interbraided in first and second sets of helices, running in opposite directions about a common longitudinal axis, and parallel to form a first braid angle with the latticework in its nominal state. The textile strands of the sleeve preferably are similarly braided in two sets of oppositely directed helices and at a second braid angle with the sleeve in its nominal state. The first and second braid angles preferably are within about 5 degrees of one another, and more preferably are within 3 degrees of each other. In a helical weave, the braid angle is an important factor in determining the degree of radial reduction for a given amount of axial elongation. Thus, the latticework and sleeve, having substantially similar braid angles and substantially the same size and shape in their nominal states, behave according to substantially the same relationship of radial reduction versus axial elongation. Accordingly, there is no tendency in the latticework to tear free of the sleeve due to its more rapid axial elongation for a given radial reduction. Conversely, there is no unwanted increase in bending stiffness due to an axial elongation of the sleeve that exceeds that of the latticework.

The latticework and sleeve are formed independently before their joinder. Structural strands forming the latticework and textile strands forming the sleeve are wound helically about respective mandrels at their respective braid angles, then thermally set, which in the case of metallic structural strands includes age-hardening. The latticework and sleeve are joined to one another with a curable adhesive, preferably a siloxane polymer. The polymer may be dissolved in a liquid organic solvent and applied to the latticework as a spray that leaves a silicone coating or residue when the solvent evaporates. The coated latticework is radially compressed and inserted axially within the sleeve, then expands to engage the sleeve. The bond is completed by heating the latticework and sleeve sufficiently to cure the adhesive. Alternative adhesives include the polycarbonate urethanes disclosed in U.S. Patent No. 5,229,431 (Pinchuk).

There are several approaches to matching the respective braid angles. In one approach the latticework is formed on a mandrel smaller than the mandrel used for the sleeve, and the structural strands and textile strands are wound at the same braid angle. Alternatively the respective mandrels can have the same diameter. Then, the textile strands are wound at a braid angle slightly less than that of the structural strands. Then, as the sleeve undergoes the slight radial enlargement necessary for accommodating (surrounding) the latticework in the nominal state, its braid angle increases toward a closer match with the braid angle of the latticework.

Further in accordance with the present invention, there is provided a body insertable stent graft as claimed in claim 20. The stent graft includes an expandable stent formed of a plurality of interconnected structural strands, adjustable between a nominal state and a radially-reduced axially-elongated delivery state. The stent graft also includes a tubular sleeve formed of a plurality of interwoven textile strands, adjustable between a nominal state and a radially-reduced and axially-elongated delivery state. Each of the textile strands is a multifilament yarn in which the multiple filaments have a surface twist angle of at most about 15 degrees. The sleeve and the latticework have substantially the same radii when in their respective nominal states. An attachment component fixes the latticework and the sleeve together, in a selected axial alignment with one another, engaged with one another and with a selected one of the latticework and sleeve surrounding the other, whereby the latticework structurally supports the sleeve.

According to another aspect of the invention, the yarns are formed to define a non-circular cross-section with major and minor axes, with an aspect ratio (major axis:minor axis) of at least about 2.

The flatter yarns with substantially untwisted filaments provide a fabric sleeve improved in the following respects: elongation behavior that more closely matches the elongation behavior of the stent; thinner walls for a reduced delivery profile; smaller interstices between yarns achieve lower permeability; and higher yarn cross-section porosity to allow tissue ingrowth.

Although the sleeve surrounds the latticework in the more preferred approach, an alternative construction features a sleeve surrounded by the latticework. In this latter case, the sleeve should be formed on a mandrel as large as the mandrel used to form the latticework, promoting a better bond with a sleeve that tends to elastically radially expand against the surrounding latticework. According to another alternative, two polymeric sleeves are employed, with the latticework sandwiched between an exterior sleeve and an interior sleeve. A variety of enhancements are provided within the scope of the present invention, e.g. incorporating one or more radiopaque strands in the latticework or sleeve, incorporating bioabsorbable materials, providing axial runners to enhance resistance to radial compression, and coating the completed stent graft or individual strands, to reduce deployment forces and lower the inflammatory response of tissue to the implanted device.

Thus in accordance with the present invention, a stent graft incorporates a structural latticework and low-permeability sleeve, independently formed and integrally connected to simultaneously provide the structural advantages of a stent and the low permeability of a graft. The latticework and sleeve are matched to undergo substantially the same degree of radial contraction for a given axial elongation. This matching, combined with an adhesive bond of the sleeve to the latticework, ensures that the stent graft radially expands and contracts as a unitary body, despite being composed of different structural and textile layers and despite the absence of an interweaving between the different layers or between the different types of strands.

IN THE DRAWINGS

For a further appreciation of the above and other features and advantages, reference is made to the following detailed description and to the drawings, in which:

Figure 1 is a side elevation, partially in section, showing a stent graft constructed in accordance with the present invention contained within a deployment device;

Figures 2 and 3 illustrate the stent graft in an unconstrained, radially expanded state, in side and end elevation, respectively;

Figure 4 is an enlarged view of the stent graft, showing the interbraiding of several textile strands;

Figures 5-9 schematically illustrate fabrication of the prosthesis;

Figure 10 shows the stent graft of Figure 2 deployed within a vessel and spanning an aneurysm;
Figure 11 illustrates an alternative embodiment stent graft with interior and exterior sleeves;
Figure 12 illustrates another alternative stent graft with localized bonding of its latticework and sleeve;
Figure 13 illustrates another alternative stent graft incorporating auxiliary strands;
Figure 14 illustrates an alternative embodiment stent graft with flexible axial strands;
Figure 15 illustrates an alternative embodiment stent graft with a plastically deformable latticework;
Figure 16 illustrates the stent graft of Figure 15 mounted on an alternative deployment device;
Figure 17 shows a further alternative stent graft with selectively positioned sleeves;
Figure 18 is a cross-sectional view of a multifilament yarn used in forming a fabric graft according to the invention;
Figure 19 is a side elevation of a segment of the yarn; and
Figure 20 is an end elevation view of a stent graft in an unconstrained, radially expanded state.

DETAILED DESCRIPTION OF THE PREFERRED EMBODIMENTS

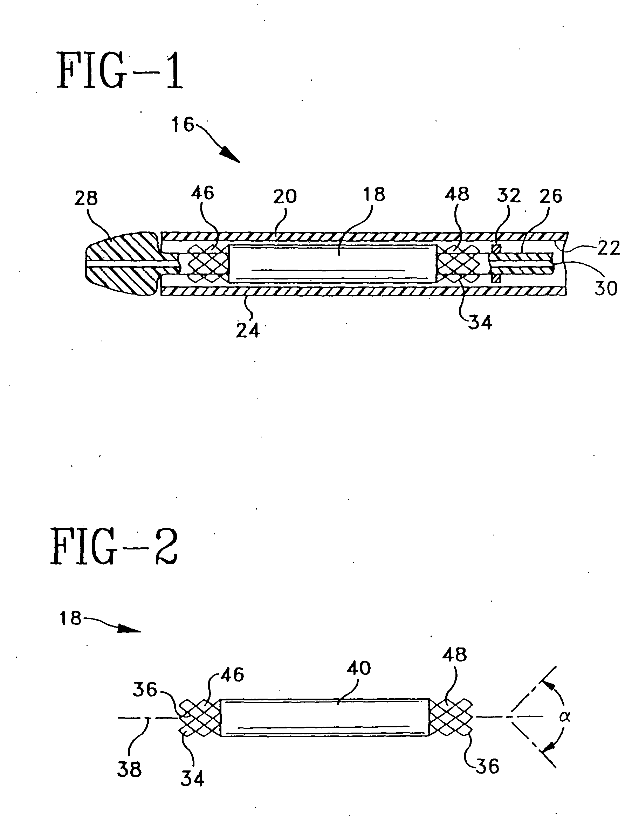

Turning now to the drawings, there is shown in Figure 1 a deployment device **16** for delivering a stent graft **18** to an intended fixation site or treatment site within a body lumen, then controllably releasing the stent graft for radial self-expansion and fixation within the lumen.

The device includes an elongate and flexible outer catheter **20** constructed of a biocompatible polymer such as polyurethane. A central lumen **22** runs the length of catheter **20**. A distal end region **24** of the outer catheter surrounds stent graft **18**. An inner catheter **26** is contained within lumen **22** and runs along the entire length of the outer catheter. At the distal end of inner catheter **26** is a tapered distal tip **28** which extends beyond the outer catheter. Stent graft **18** surrounds inner catheter **26**, confined between the inner and outer catheters. A lumen **30** in the inner catheter can accommodate a flexible guidewire (not shown) tracked by device **16** as it is advanced toward the treatment site.

Stent graft **18** is formed of resilient materials, and in Figure 1 is shown elastically compressed into a radially-reduced and axially-elongated delivery state. Outer catheter **20** maintains the stent graft in the delivery state against its elastic restoring force. An annular detent **32**, mounted to inner catheter **26**, occupies a space between the inner and outer catheters to limit proximal travel of the stent graft relative to the inner catheter. As outer catheter **20** is moved proximally relative to inner catheter **26**, the detent prevents the stent graft from following the outer catheter.

Catheters **20** and **26**, while maintaining stent graft **18** in the delivery configuration, are moved transluminally to deliver the stent graft to the treatment site. Once the stent graft is positioned as intended, inner catheter **26** is held stationary while outer catheter **20** is withdrawn proximally. Inner catheter **26,** due to detent **32,** maintains the stent graft property aligned as it progressively radially self-expands toward an intimate contact with tissue at the treatment site. The stent graft does not expand completely to the relaxed state, and thus exerts a residual force on surrounding tissue that tends to acutely fix the prosthesis. At this point the stent graft has a diameter much larger than the diameter of distal tip **28**, so that the inner catheter and tip are proximally withdrawn to leave the stent graft in place.

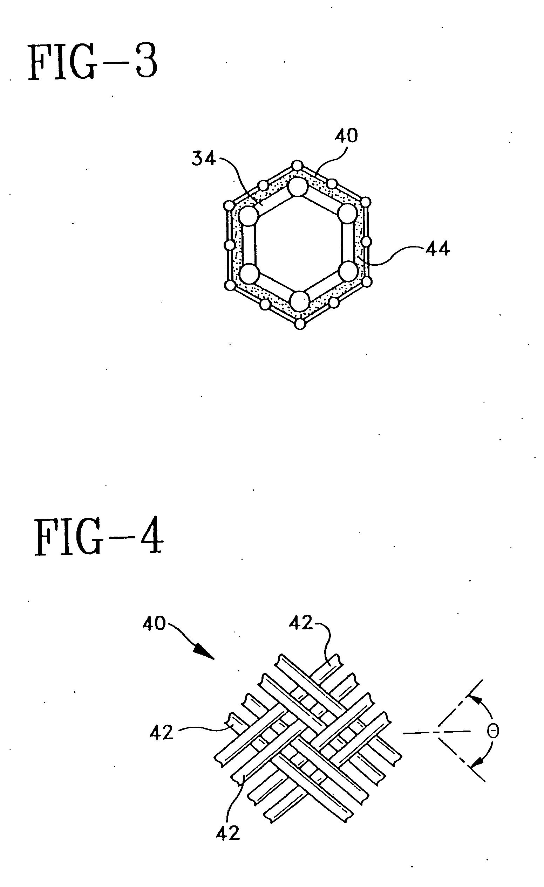

Stent graft **18** in Figures 2 and 3 is shown in a nominal state, in this case a relaxed state in which the stent graft is subject to no external forces. As compared to the delivery state, the prosthesis has a substantially enlarged radius and a substantially reduced axial length. The stent graft has several concentric layers which act in concert during radial expansions and contractions.

The inside layer (radially) is a latticework **34** formed of resilient monofilament structural strands **36**. Strands **36** are arranged in two sets of parallel helices wound in opposite directions about a common longitudinal axis **38**. Strands **36** intersect one another to define rhombotic interstices and a braid angle $\alpha$ bisected by axis **38**.

The braid angle is defined with reference to the nominal state of the latticework. As seen from Figure 1, radial compression of the stent graft into the delivery state substantially reduces the braid angle. The braid angle largely determines the relationship between radial compression and axial elongation of the prosthesis. Smaller braid angles result in less axial shortening for a given amount of radial enlargement. Conversely, given a larger braid angle, the same radial expansion results in more axial shortening. For a given strand size and strength, a larger braid angle imparts greater resistance to radial compression and more positive acute fixation.

Monofilament structural strands **36** can be formed of polymers as well, including PET, polypropylene, PEEK, HDPE, polysulfone, acetyl, PTFE, FEP, and polyurethane. Suitable diameters for the monofilament strands range from about 0.002 inches to about 0.015 inches (0.05 to 0.38 mm)

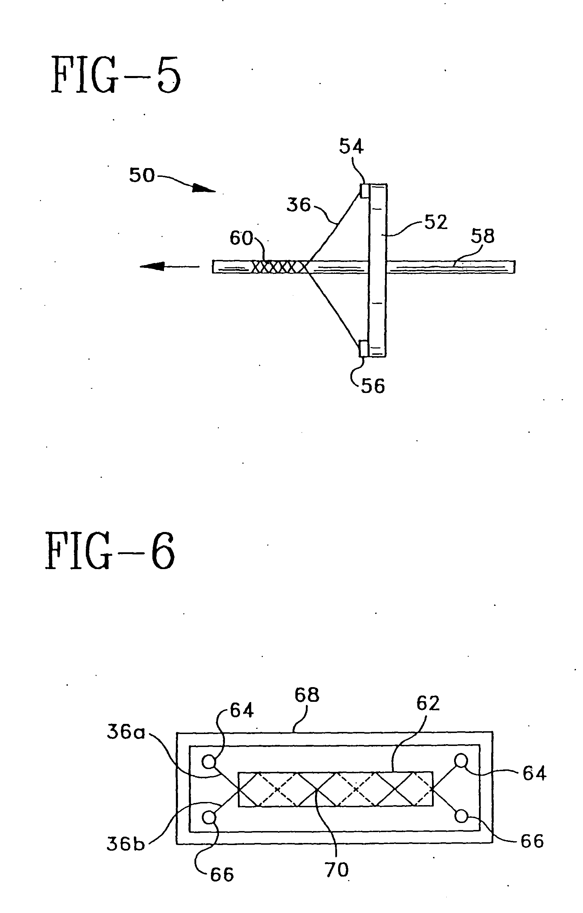

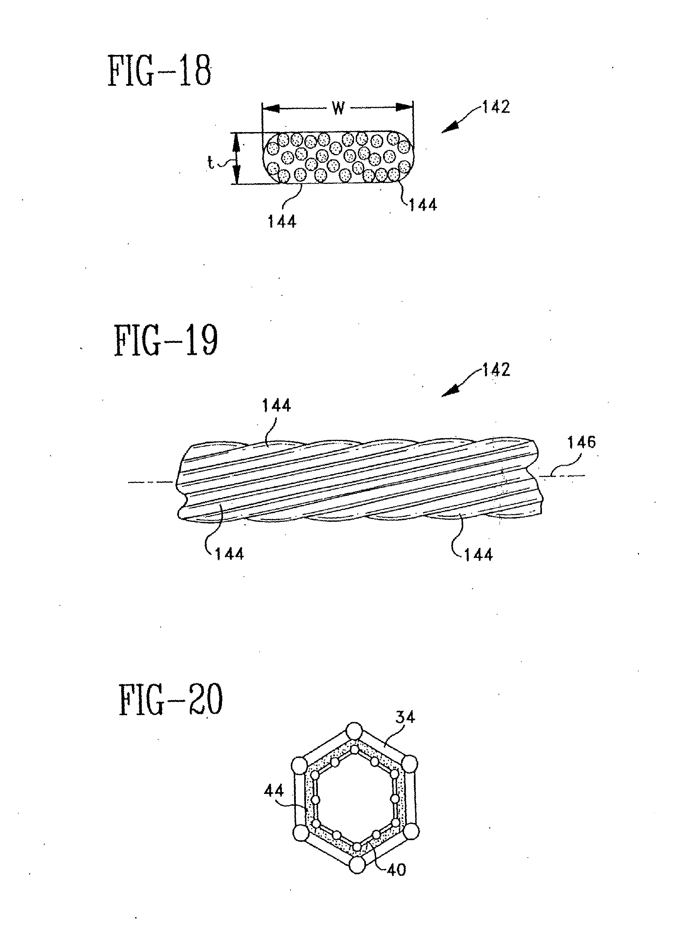

Figure 3 shows an exterior layer of stent graft **18** as a textile sheeting or sleeve **40**, formed of multiple textile strands 42 interwoven with one another. Figure 20 shows an alternative embodiment wherein sleeve **40** is an interior layer. As seen in Figure 4, the textile strands are braided in a two over two pattern. although a one over one pattern or other patterns are suitable as well. The textile strands intersect one another to define a braid angle $\theta$ with sleeve **40** in a nominal state. Braid angle $\theta$, like angle $\alpha$, is bisected by axis **38.** The number of yarns in the polymeric sleeve can range from 20 to 700.

Textile strands **42** preferably are multifilament yarns, although they can be monofilaments. In either case the textile strands are much finer than the structural strands, ranging from about 10 denier to 400 denier. Individual filaments of the multifilament yarns can range from about 0.25 to about 10 denier. The multifilament yarns generally have a high degree of compliance, which may or may not include elasticity. Preferably the multifilament yarns are composed of PET (Dacron). Other acceptable materials include polypropylene, a high molecular weight polyethylene sold under the brand name "Spectra", polyurethane, HDPE, polyethylene, silicone, PTFE, polyolefins and PTFE.

Further, it is preferable to form the multifilament yarns, such that the multiple filaments are substantially untwisted and cooperate to form an oblong or non-circular yarn cross-section. These features, and the resulting performance advantages in stent grafts that incorporate them, are discussed in more detail below, in connection with Figures 18 and 19.

Because of the fineness of the textile strands and close or tight weave, sleeve **40** can be microporous, yet essentially impervious to body fluids. The textile sheeting of the sleeve is highly compliant, conforming to changes in the shape of latticework **34** as stent graft **18** either radially self-expands or is radially compressed. The shape of the latticework determines the shape of the stent graft.

Despite the compliant nature of sleeve **40,** proper matching of the sleeve and latticework **34** is critical to achieving high performance. In broad terms, the latticework and sleeve are matched so that when adjusted between their respective nominal and delivery states, they behave according to the same relationship of radial reduction versus axial elongation. This relationship can be expressed in terms of a percentage of radial contraction for a given percentage of axial elongation, or more succinctly a ratio of contraction to elongation. Such ratio varies with the braid angle. More particularly, at higher braid angles a given radial contraction produces a greater axial extension. In this embodiment, appropriate matching involves forming latticework **34** and sleeve **40** with the same braid angle, i.e. $\theta$ equals $\alpha$. Satisfactory matching occurs if $\theta$ is within 5 degrees of $\alpha$, more preferably if $\theta$ is within 3 degrees of $\alpha$. A highly preferred matching involves selecting angles $\alpha$ and $\theta$ within one degree of each other. In different embodiments, appropriate matching may require a difference in angles $\theta$ and $\alpha$. For example, where a sleeve is intended to surround a latticework, it may be advantageous to form the fabric sleeve with a braid angle $\theta$ less than the corresponding braid angle $\alpha$ of the latticework.

In embodiments such as illustrated in Figure 3 where graft **40** is disposed about structural support **34**, the braiding angle of textile strands **42** is preferably between 100.91 and 105.45 percent of the braiding angle of structural strands **36**. In embodiments such as illustrated in Figure 20 where graft **40** is disposed inside structural support **34**, the braiding angle of textile strands **42** is preferably between 97.72 and 102.27 percent of the braiding angle of structural strands **36**.

Latticework **34** and sleeve **40** are integrally secured to one another by an adhesive layer **44**, more particularly silicone. The silicone adhesive forms an extremely thin (e.g. 0.005 inches, 0.12 mm) between the latticework and sleeve, adhering to both. Due to the matching of the latticework and sleeve braid angles, adhesive layer **44** is required to accommodate only slight movement of the sleeve relative to the latticework during radial expansions and contractions of the stent graft. Matching avoids a difference in rates of axial elongation that otherwise would unduly stress the adhesive layer and/or cause unwanted stiffness in resistance to bending. As a result, the stent graft is usable over a wider range of radii, elastically compressible to a smaller radius, and able to negotiate more tortuous or serpentine arterial passages.

Latticework **34** is longer than sleeve **40** and extends beyond the opposed ends of the sleeve, providing respective open-mesh end portions **46** and **48** to facilitate acute and long-term fixation. In other embodiments, latticework **34** and sleeve **40** will be congruent in length.

Stent graft **18** is fabricated according to several steps as illustrated in Figures 5-9. Figure 5 schematically illustrates a braiding apparatus **50**, including an annular carrier assembly **52**. The carrier assembly supports multiple bobbins in a circular array, with two of the bobbins indicated at **54** and **56**. The apparatus further includes a cylindrical mandrel **58**, centered within the carrier and movable longitudinally relative to the carrier as indicated by the arrow.

In forming latticework **34**, carrier assembly **52** is loaded by winding structural strands **36** onto the bobbins. The structural strands are drawn from their respective bobbins to mandrel **58** and braiding proceeds by moving the mandrel longitudinally, while at the same time the bobbins are moved relative to one another as dictated by the desired braiding pattern. The result is an interbraiding of structural strands onto the mandrel in two oppositely directed sets of helices, as indicated at **60**. The mandrel determines the diameter of the braided structure. Mandrel longitudinal speed determines the braid angle. The latticework lengths are determined by the duration of braiding, or by cutting the braided structure to desired lengths after its removal from the mandrel.

After their removal from mandrel **58**, the structural strands are heat treated to determine the shape of the latticework in its nominal state. Figure 6 illustrates two structural strands (metal monofilaments) **36a** and **36b**, one from each set of oppositely directed structural strands, wound about a shaping mandrel 62 and supported by respective bobbins **64** and **66.** While just strands **36a** and **36b** are illustrated, it is to be appreciated that all of the structural strands are maintained together about the mandrel for shaping.

For metallic monofilaments, the heat treatment involves age-hardening within a furnace **68** in a vacuum or a protective atmosphere. Temperatures are within the range of about 350-1000 degrees C., with the specific temperature depending on the structural material involved. The monofilaments overlie one another to form multiple intersections, one of which is indicated at **70**. The bobbins, including bobbins **64** and **66**, are set to tension their respective strands during age-hardening. The appropriate duration for age-hardening varies with materials and dimensions, but can range from as brief as thirty seconds, to about five hours.

As they cool after age-hardening, structural strands **36** retain their helical shapes and collectively determine the nominal or relaxed state of latticework **36**. In this embodiment the monofilament structural strands are highly resilient, i.e. deformable under external stress but elastically returning to the nominal state when free of the external stress.

When structural strands **36** are thermoplastic rather than metallic, multiple strands are thermally set in similar fashion, but at lower temperatures. Heat forming temperatures typically range from about 100 to about 400 degrees C., and more preferably 150 to 250 degrees C. The strands are maintained at or above the heat forming temperature for a duration generally shorter than that of thermally setting metal strands, i.e. from about thirty seconds to about two hours, or more preferably five to fifteen minutes.

Sleeve **40** is formed by braiding textile strands **42** about a mandrel, again in two sets of parallel, oppositely directed helices. Braiding apparatus **50** or a similar device can be used. The multifilament yarns can be wound about mandrel **58**, or another mandrel with a slightly larger diameter. A primary consideration is to select braid angle $\theta$ with respect to braid angle $\alpha$ of the latticework, to closely match the geometrical diameter and elongation properties of the latticework and sleeve when these components are formed into the stent graft. With latticework **34** and sleeve **40** both composed of helically wound strands, this is accomplished in most cases by matching the respective braid angles, with matching on occasion involving a slight difference in the braid angles, as noted above.

Accordingly, if textile strands are wound about mandrel **58** (or another mandrel of the same diameter), braid angle $\theta$ is intentionally set a few degrees less than braid angle $\alpha$. When sleeve **40** surrounds latticework **34** in the finished stent graft, it necessarily is expanded to a slightly larger radius, which entails increasing braid angle $\theta$ toward coincidence with braid angle $\alpha$.

Alternatively, textile strands **42** can be braided at the same braid angle as the latticework, but on a larger diameter mandrel to account for the fact that sleeve **40** surrounds latticework **34** and the intermediate adhesive. As a final alternative these approaches can be combined, with the textile strands braided at a slightly reduced braid angle on a slightly larger mandrel.

In any event, the textile strands are thermally set while wound about a shaping mandrel, to give sleeve **40** its nominal state. The multifilament yarns are thermally set in substantially the same manner as the thermoplastic structural strands. Typically the multifilament yarns are thermally set at temperatures ranging from 150 to 250 degrees C., for times ranging from 1 to 30 minutes. More preferably, setting temperatures range from 180 to 210 degrees C., and the time for setting is about 3-5 minutes.

After the sleeve is thermally set, it is removed from the mandrel and washed ultrasonically to remove any finish on the yarn. Then, sleeve **40** is cut to the desired length using a laser, which introduces localized fusing at the ends of the strands to prevent unraveling.

Thus, prior to their joinder the latticework and sleeve are formed independently of one another. This is advantageous from the standpoint of allowing braiding and thermal setting conditions to be tailored specifically in each case to the strand structure and material involved.

Next, the completed sleeve and latticework are bonded to one another. Preferably the bond extends circumferentially about the interface between the latticework exterior surface and sleeve interior surface, over the complete axial length of the annular region over which the sleeve and latticework are adjacent one another. Further, the bond should be highly uniform over the entire region, to avoid stress concentrations and ensure a more uniform behavior of the stent graft during bending, radial expansions and radial contractions.

To this end, a siloxane polymer (silicone) is used as the adhesive and is applied as a uniformly thick coating to latticework **34**. To ensure a more uniform coating, the silicone is dispersed in an organic solvent of xylene and THF (tetrahydrafuran) at a concentration of about 6% of the silicon polymer, by weight. Suitable alternative organic solvents include toluene and trichloroethylene. Suitable alternative polymers include fluorosilicone and polycarbonate urethane. Alternative adhesives include polycarbonate urethanes such as disclosed in U.S. Patent No. 5,229,431 (Pinchuk).

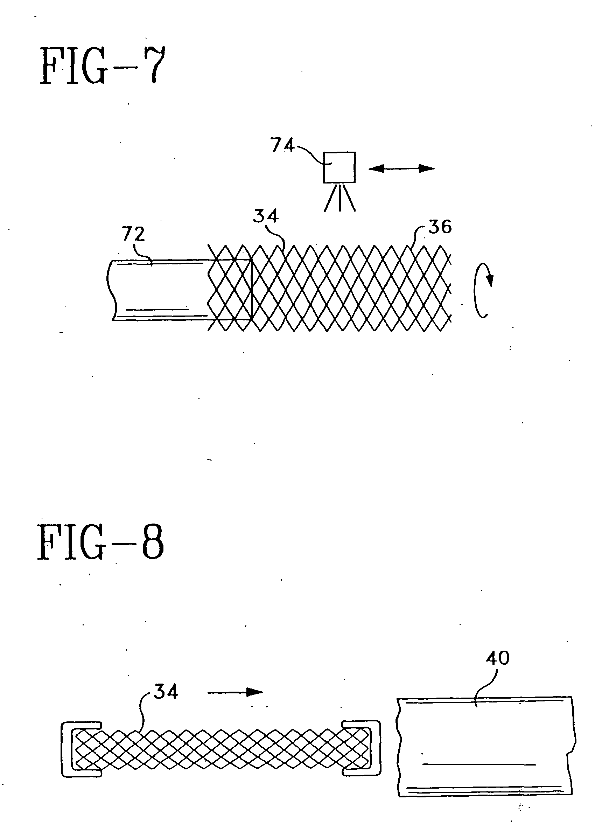

As seen in Figure 7, latticework **34** is placed in a fixture **72** that rotates the latticework about axis **38** at a speed of about 100 rotations per minute. Then, a spraying device **74** (e.g. an air brush) is used to spray the polymer/solvent solution onto the latticework, to a uniform thickness of about 0.005 inches over the entire latticework surface. The xylene and THF are allowed to evaporate, leaving as a residue the silicone polymer, thus forming a uniform adhesive coating over the latticework.

As an alternative, a polymeric adhesive in powdered form, e.g. polypropylene or polyethylene, can be electrostatically deposited onto the latticework according to a technique known to those skilled in the art. The polymer when applied is kept below its melting point, although it may reach a fusion or glass transition temperature. Later, during bonding of the latticework and sleeve, the polymer is heated at least to its melting temperature.

As another alternative, the adhesive can be applied by a hot melt process, in which a polymer (e.g. polyurethane) is applied to the latticework in liquid form.

Next, the latticework and sleeve are assembled by axially elongating the latticework to reduce its radius, then inserting the reduced-radius latticework into sleeve **40** while the sleeve is maintained in its nominal state. Then, the silicone-coated latticework is allowed to axially shorten and radially expand into a surface engagement with the radially inside surface of sleeve **40**. The thickness of the silicone coating is taken into account in the sizing of the sleeve and latticework with respect to one another. It has been found advantageous to select the respective radii of sleeve **40** and latticework **34** so that when allowed to expand, the latticework exerts a slight radial elastic force onto the surrounding sleeve, with the sleeve preferably exerting a counteracting radially inward force upon the latticework. These counterbalancing forces improve the bond.

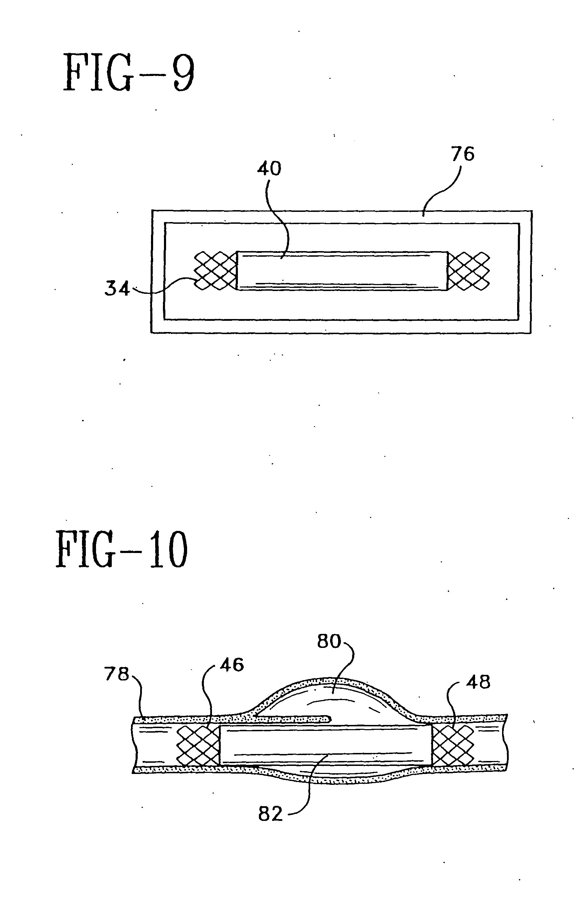

At this stage it is necessary to cure the silicone polymer adhesive. For this purpose, the stent graft is maintained in an oven **76** (Figure 9) at temperatures in the range of 125-200 degrees C., for a time ranging from 20 minutes to about one hour. More preferably, the temperature is about 150 degrees C. and the time is about 30 minutes. After curing, the stent graft is removed from the oven and allowed to cool to ambient temperature. The cured silicone polymer adheres to sleeve **40** as well as the latticework, causing stent graft **18** to behave as a unitary structure, notwithstanding its three distinct layers **34, 40**, and **44** as discussed above.

Thus formed, stent graft **18** combines the favorable attributes of self-expanding stents and grafts. Latticework **34** provides radial compressibility, self-expansion over a wide range of radii and a residual force sufficient for acute fixation, while sleeve **40** provides reduced permeability so that the stent graft is essentially impervious to blood and other body fluids. As a result the stent graft is particularly well suited for treating an aneurysm. Figure 10 illustrates fixation of stent graft **18** within a blood vessel having a vessel wall **78**. Along the vessel wall is an aneurysm **80.** Opposite end portions **46** and **48** of the stent graft are radially expanded into intimate contact with vessel wall **78** on opposite sides of the aneurysm. A medial region **82** of the stent graft spans the aneurysm. End portions **46** and **48** effectively fix the stent graft, due to the resilience and strength of the structural strand latticework. Fixation is enhanced by the exposed end portions. Because of the open weave latticework structure, end portions **46** and **48** more effectively engage surrounding tissue for a better acute fixation. The open structure also promotes fibrotic ingrowth, which is desirable near the end portions because it improves chronic fixation.

The following examples concern fabrication of stent grafts within the scope of the present invention.

Example 1:

The latticework is produced by setting up a 48 carrier braiding apparatus in a one over one diamond braid pattern, using 24 of the carriers. The corresponding 24 bobbins are loaded with a 0.13 mm (0.0055 inch)diameter Elgiloy wire. A 10.0 mm diameter round mandrel is placed onto the puller of the braiding device. The Elgiloy wires are threaded through their respective carriers and attached to the mandrel. The braiding apparatus is set to form a braid angle of 110 degrees. When an adequate length of the braid is formed, the latticework is removed from the mandrel. Then, the latticework is mounted onto a shaping mandrel and heat treated in a vacuum furnace, increasing the strength of the Elgiloy strands as well as inducing a more permanent memory into the strands to set the normal state of the latticework.

The polymeric sleeve is formed on a 192 carrier braiding apparatus, set in a two over two braid pattern using all 192 carriers. The corresponding bobbins are loaded with a 40 denier, 27 filament polyester yarn. A 10.5 mm diameter mandrel is inserted into the puller. The braider is operated to form a braid angle of 107 degrees. When a sufficient length of the sleeve is formed, the sleeve is removed from the braiding mandrel. Next, the sleeve is thermally set on a shaping mandrel at a temperature of 205 degrees C. for about 5 minutes. Next, the sleeve is ultrasonically cleaned to remove any finish on the yarn. The sleeve length is determined by laser cutting at the opposite ends to prevent unraveling.

An uncured silicone polymer is applied to the Elgiloy latticework, sprayed onto the latticework as a liquid solution with the silicone at six percent by weight. The silicone solution is sprayed onto the latticework at an air pressure of about $10.4 \times 10^3$ Pa (15 psi.) Spraying occurs intermittently in sets of several (preferably three) passes of the airbrush sprayer, allowing several seconds for settling between sets of airbrush passes, until an amount of solution corresponding to a desired thickness has been sprayed, i.e. approximately 26 ml for the 10 mm diameter latticework. Alternatively, the silicone solution can be applied in a continuous spray, typically lasting about 5 minutes.

The coated latticework is allowed to air dry to a tacky condition, then constrained to an axially elongated reduced radius state for insertion into the completed sleeve. Grips holding opposite ends of the latticework are moved towards one another slowly, to effect a gradual return of the latticework toward its nominal radius, and toward engagement with the surrounding sleeve. Once the latticework is completely released and engaged with the sleeve, the stent graft is cured at 150 degrees C. for thirty minutes.

Example 2:

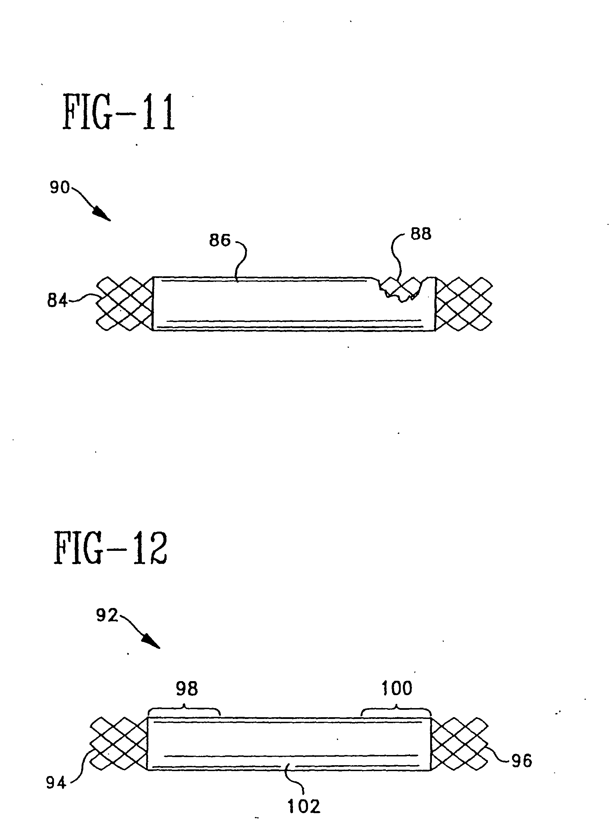

A latticework **84** and surrounding polyester sleeve **86** are formed as before. Further, a second polyester sleeve **88**, for use as an interior sleeve surrounded by the latticework, is braided on a 10.0 mm mandrel, with the braider operated to form a braid angle of 107 degrees. The latticework is axially elongated and inserted into the exterior sleeve, then allowed to gradually radially expand against the sleeve as before. Then, the second, inner polyester sleeve is axially elongated, placed within the latticework and exterior sleeve, and radially expanded into engagement against the latticework. A 9.5 mm mandrel, inserted into the interior sleeve, further urges the sleeve radially outward and into contact with the latticework. The stent graft, including the latticework and the interior and exterior sleeves, is maintained at 150 degrees C. for thirty minutes to cure the silicone adhesive. The resulting stent graft 90 is shown in Figure 11.

Example 3:

A latticework and exterior polyester sleeve are formed as before. The latticework is sprayed with a silicone solution as before, but only along two opposite end regions, leaving a medial segment of the latticework uncovered. The latticework was axially elongated, inserted within the sleeve and allowed to radially expand against the sleeve, as in the previous examples. Oven curing was substantially the same.

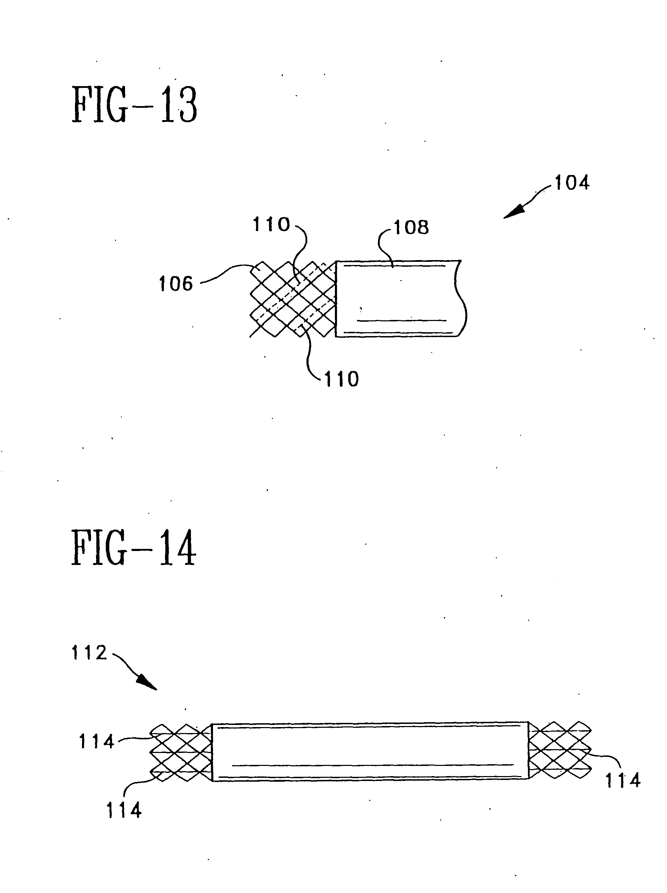

The result, shown in Figure 12, is a stent graft **92** with exposed ends **94** and **96** of the latticework coated with silicone. Opposite end bond regions **98** and **100** have axial lengths of about 17 mm, where the latticework and sleeve are bonded together. Over a medial region **102**, the sleeve and latticework are adjacent one another and in surface contact, but not bonded. Where the stent graft is provided to an end user in elongated tubular form, with the intention that the end user will cut the tube to the desired lengths, bonding along the full lengths of the latticework and sleeve is recommended.

Stent grafts can be fabricated to impart a variety of desired qualities. For example, Figure 13 shows a stent graft **104** formed with a latticework **106** of structural strands surrounded by a sleeve **108** of textile strands. An auxiliary strand **110** is interbraided with the textile strands. Strand **110** can be formed of a radiopaque material, e.g. tantalum, to improve fluoroscopic imaging of the stent graft. Alternatively, biological or bioabsorbable strands can be interwoven in this fashion.

Figure 14 illustrates a stent graft **112** in which polyurethane monofilaments have been incorporated into the latticework as axial runners **114**. To form the axial runners, the braiding apparatus is provided with an appropriate number of triaxial guide tubes arranged about the carrier assembly. One of the polyurethane monofilaments is fed into each of the triaxial guide tubes. During the curing stage, the polyurethane runners are heated sufficiently to fuse them to the remaining structure. The axial runners enhance radial recovery of the graft and reduce the tendency to unravel or fray.

Another improvement, not illustrated, is a coating of the multifilament yarn textile strands with TFE (tetrafluoroethylene) or a copolymer of TFE and silicone. The coating, which can be applied by plasma polymerization before braiding the sleeve, enhances surface properties of the yarn, e.g. by reducing friction. The resulting stent graft can be deployed with less force and inflammatory responses to the implanted stent graft may be reduced. Alternatively a plasma polymerization can occur after fabrication of the stent graft, to coat the exterior surface of the sleeve.

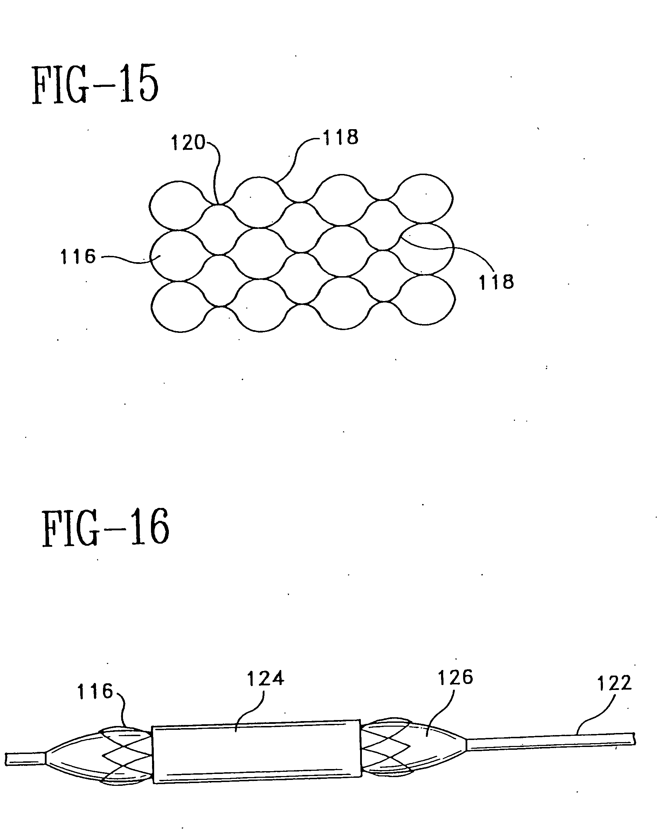

Figure 15 discloses an alternative latticework **116**, formed by a series of plastically deformable sinusoidal strands **118** joined to one another at multiple points **120**. Latticework **116** can be plastically adjusted by a combined radial reduction and axial elongation. Strands **118** are metallic, but formed of metals much more ductile and malleable than the metals forming latticework **34** (for example). As an alternative, latticework **116** can be formed of plastically deformable strands arranged in sets of oppositely directed helices.

Figure 16 illustrates the distal end region of a catheter **122** used to deploy a stent graft **124** including latticework **116**. Stent graft **124** is governed by the structural characteristics of latticework **116** and thus remains in the axially elongated radially reduced delivery state as shown, without an additional catheter or other constraining feature. The stent graft has no tendency to elastically resume its larger radius nominal state. Thus, an auxiliary forcing feature is required to urge the stent graft, once properly positioned, toward its normal state. For this purpose a dilatation balloon **126** is mounted to catheter **122** and surrounded by the stent graft. The balloon, when inflated by the introduction of fluid under pressure through a lumen in catheter 122, radially expands the stent graft.

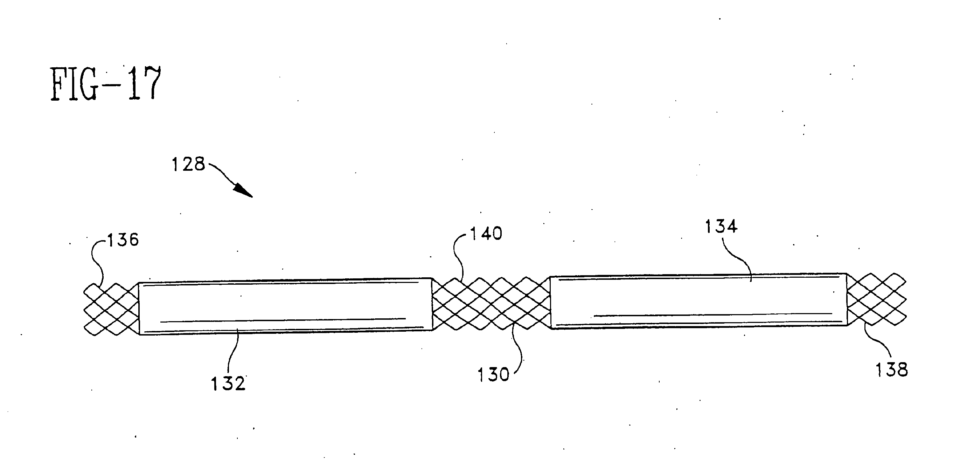

Figure 17 shows a stent graft 128 with a latticework 130 surrounded by proximal and distal sleeves **132** and **134.** The latticework is exposed at stent graft end portions **136** and **138,** for improved acute and long-term fixation as explained above. Each of sleeves **132** and **134** is positionable along an intraluminal location where shunting of the blood flow is desired. An exposed medial region **140** between sleeves **132** and **134** is positionable in alignment with a branch of the vessel being treated, so that stent graft **128** can provide the intended shunting without blocking flow between the main vessel and the branch between the two shunting areas.

Stent graft **128** is fabricated according to the process discussed above, i.e. with the latticework and sleeves being independently braided, then bonded with a silicone adhesive over the cylindrical regions along which the sleeves and latticework are adjacent, i.e. along the axial lengths of the sleeves. Sleeves **132** and **134,** or any number of sleeves, are bonded to the latticework in a single curing operation. Sleeves **132** and **134** can be cut from the same braiding of textile strands, or braided independently, for example if different diameter sleeves are desired. In any event a stent graft can be tailored to meet specific needs by altering variables such as the number of sleeves employed, the diameter and axial length of the sleeves, and their positioning along the latticework.

As previously noted, the multifilament yarns used in commercially available textile vascular grafts are twisted yarns, i.e. the multiple filaments making up the yarn have surface twist angles in the range of about 15 degrees to about 45 degrees. The multiple filaments characteristically define a circular yarn cross-section. This structure has been favored, because such yarns are structurally stable and lend themselves well to handling by the equipment used in weaving and knitting processes. With respect to stent grafts, especially of the self-expanding type, this yarn structure is a detriment to stent graft performance in several respects.

The high degree of filament twisting can cause kinking of the fabric when the stent graft is bent, which limits the curvature of vessels in which the stent graft can be deployed and implanted. In twisted yarns the multifilaments are tightly packed, with the packing factor (ratio of cross-sectional area of the combined filaments to the cross-sectional area of the yarn as a whole) in the range of 0.7-0.9. Thus the void, or the accumulation of interstices between adjacent filaments, is insufficient for tissue ingrowth. Fibrotic ingrowth is desired, because it contributes to an effective chronic fixation of the stent graft.

The tight packing of the filaments and circular yarn cross-section combine to unnecessarily limit the elongation capabilities of the fabric graft, because the tightly packed yarn lacks the capability of adjusting itself in cross-sectional profile during elongation.

The circular cross-section sets a minimum dimension for the stent graft, in that the fabric graft wall is preferably at least as thick as two yarn diameters. The fabric coverage is undesirably low because of the circular yarn cross-section, i.e. usually below 80 percent without additional compacting. The fabric porosity is undesirably high, usually above 70 percent without additional compacting.

The above disadvantages are overcome according to a preferred embodiment of the present invention, namely construction of the graft or sleeve with an essentially untwisted (or slightly twisted) multifilament yarn in which the filaments have a surface twisting angle of at most about 15 degrees. Further, the yarn is formed with a preferred, non-circular cross-section. As seen in Figure 18, a textile strand or yarn **142** is composed of multiple filaments **144** arranged to define a yarn cross-sectional shape with an aspect ratio slightly greater than three. In more general terms, the aspect ratio is defined as:

$$f = w/t$$

where f is the aspect ratio, w is the cross-sectional width, and t is the cross-sectional thickness. The values w and t also can be thought of as respective major and minor axes of the yarn cross-section. The yarn as shown in Figure 18 consists of untwisted fibers having circular cross-sections. In cases where the fibers are twisted, their cross-sections would appear elliptical. While satisfactory non-circular cross-sections can have aspect ratios up to about 20, preferred aspect ratios are in a range from about 2 to about 12.

Figure 19 illustrates a segment of multifilament yarn **142**, showing the surface twist angle of the multiple filaments that make up the yarn. Filaments **144** are helically arranged, and the surface twist angle is the angle of incline of the filament with respect to a longitudinal axis **146** of the yarn, in the same sense that braid angles $\alpha$ and $\theta$ are defined with respect to longitudinal axes of their respective tubular structures. The preferred twist angle of the filaments is at most about 5 degrees, significantly less than surface twist angles typical of conventional twisted yarns, typically with surface twist angles in the range of 15 - 45. Because of the reduced twist angle, yarn **142** can be formed with a considerably reduced packing factor. The result is more interstitial space within the yarn for fibrotic ingrowth, for more secure chronic fixation of the sleeve.

The use of multifilament yarns with the preferred non-circular cross-sections, and in which the filaments are essentially untwisted, provides several performance advantages. The lack of any substantial twisting leads to a wider range of fiber packing, with packing factors ranging from 0.5-0.9. The fiber packing factor k is defined as:

$$k = n(Af)/(Ay)$$

where n is the number of filaments or fibers in the yarn, Af is the cross-sectional area of each fiber, and Ay is the total cross-sectional area of the yarn. Selection of lower packing factors within this range, i.e. 0.5-0.7, allows the yarn cross-sectional area to change in response to changes in the stent graft; i.e. shrink in response to elongation of the fabric sleeve, and expand as the sleeve recovers toward its nominal length. Further, the more loosely packed filaments provide a more porous yarn with increased voids or interstices between filaments, enabling tissue ingrowth for improved long-term fixation of the graft.

The preferred non-circular yarn cross sections cooperate with the more loosely packed filaments to improve elongation properties. More particularly, the yarn cross-sectional shape can change, narrowing as the sleeve is elongated, then widening as the sleeve recovers towards its nominal length.

Further, the higher aspect ratio, "flattened" yarns can be made with a reduced thickness without diminishing strength, since strength is a function largely of the yarn cross-sectional area. With the yarn's thickness being less than one-third of its width, as illustrated in Figure 18, the result is a substantial reduction in thickness of the fabric sleeve. This feature, in cooperation with improved elongation capability, enables delivery of the stent graft to treatment sites in narrower vascular passages. Yet another benefit of the increased aspect ratio is the combination of better fabric coverage and reduced fabric porosity, thus reduced permeability to water and other fluids.

To summarize, the lack of any substantial filament twisting and resultant lower packing factors, and higher aspect ratios for the yarn cross sections, yield the following advantages:

1. water permeability below 2000 ml/min/cm$^2$ (at 120 mm Hg pressure), due to a yarn coverage or fabric coverage of greater than 90 percent and a porosity of less than 60 percent;
2. a substantially thinner fabric sleeve (e.g., average thicknesses less than 0.25 mm), permitting a smaller delivery profile and implantation in smaller diameter arteries; and
3. improved elongation capability in the sleeve, e.g. greater than 60 percent elongation depending on the braid angle, to better match the elongation properties of self-expanding stents.

The advantages are better understood upon consideration of the following two examples which, while based on a mathematical model, nonetheless illustrate the performance improvements realized when the fabric sleeve of a stent graft is formed with multifilament yarns having reduced filament twist, low filament packing and non-circular profiles.

Example 4:

Textile strand: a twisted, 80 denier PET multifilament yarn, having an aspect ratio of 1, and a packing factor of 0.75.

Fabric sleeve: inside diameter of 10 mm, braid angle of 110 degrees, 192 yarn ends, fabric thickness of 0.183 mm (0.0073 inches), yarn coverage of 66 percent, and fabric porosity of 76 percent.

Table 1 illustrates structural changes in the fabric sleeve and yarn, accompanying radial contraction and axial elongation of the sleeve:

Table 1

| Inner Diameter | Fabric Elongation | Braiding Angle | Fabric Thickness | Yarn Coverage | Fabric Porosity | Yarn Aspect Ratio | Fiber Packing Factor |
|---|---|---|---|---|---|---|---|
| 10.00 mm | 0% | 110N | 0.0073" | 66% | 76% | 1,0 | 0.75 |
| 9.00 mm | 18% | 95N | 0.0073" | 71% | 73% | 1.0 | 0.75 |
| 8.00 mm | 32% | 82N | 0.0073" | 72% | 73% | 1.0 | 0.75 |
| 7.00 mm | 43% | 70N | 0.0073" | 74% | 72% | 1.0 | 0.75 |
| 6.00 mm | 52% | 59N | 0.0067" | 75% | 66% | 1.0 | 0.80 |
| 5.85 mm | 53% | 57N | 0.0066" | 75% | 65% | 1.0 | 0.91 |
| 0.0073" = 0.183 mm  0.0067" = 0.168 mm  0.0066" = 0.165 mm | | | | | | | |

Example 5:

Textile strand: 40 denier PET multifilament yarn, without any substantial twisting of the filaments, with a yarn aspect ratio of 3.66 and a filament packing factor of 0.50.

Fabric sleeve: inside diameter of 10 mm, braid angle of 110 degrees, fabric thickness of 0.08 mm (0.0032 inches), yarn coverage of 95 percent, and fabric porosity of 60 percent.

Table 2 illustrates structural changes in the fabric sleeve and the yarn, as the fabric sleeve is radially contracted and axially expanded.

Table 2

| Inner Diameter | Fabric Elongation | Braiding Angle | Fabric ① Thickness | Yarn Coverage | Fabric Porosity | Yarn Aspect Ratio | Fiber Packing Factor |
|---|---|---|---|---|---|---|---|
| 10.00 mm | 0% | 110N | 0.0032" | 95% | 60% | 3.66 | 0.54 |
| 9.00 mm | 18% | 95N | 0.0032" | 96% | 63% | 3.92 | 0.50 |
| 8.00 mm | 32% | 82N | 0.0032" | 96% | 63% | 3.85 | 0.50 |
| 7.00 mm | 43% | 70N | 0.0032" | 95% | 61% | 3.66 | 0.54 |
| 6.00 mm | 52% | 59N | 0.0032" | 94% | 58% | 3.16 | 0.60 |
| 5.00 mm | 59% | 48N | 0.0032" | 92% | 53% | 2.62 | 0.72 |
| 4.00 mm | 65% | 38N | 0.0046" | 77% | 65% | 1.11 | 0.84 |
| 3.83 mm | 66% | 36N | 0.0043" | 78% | 61% | 1.15 | 0.91 |

① 0.0032" = 0.08 mm
0.0046" = 0.115 mm
0.0043" = 0.108 mm

As is clear from comparing Table 2 with Table 1, the use of essentially untwisted multifilament yarns defining non-circular yarn cross-sections considerably improves the resulting fabric sleeve. Axial elongation is 66 percent in the preferred sleeve, compared to 53 percent elongation in the sleeve composed of conventional, twisted yarn. The inner diameter at full elongation is substantially less for the preferred sleeve, i.e. 3.83 mm as compared to 5.85 mm. Thus, the preferred sleeve, when elongated to its reduced-radius delivery state, can travel through much narrower vascular passages, and enables use of a smaller profile delivery device.

The preferred device has a much thinner fabric wall, both in the nominal state (no axial expansion) and when axially expanded. This augments the improved elongation capability, to further reduce the diameter of the sleeve in the delivery state.

In the preferred sleeve, the yarn coverage or fabric coverage is significantly higher (95 percent versus 66 percent), and the fabric porosity is significantly lower (60 percent versus 76 percent), resulting in lower permeability.

Because the filaments in the preferred yarn are less tightly packed (packing factor before extension of .54 versus .75), porosity on a "micro" scale, i.e. throughout the yarn cross-section, is substantially greater, resulting in more tissue ingrowth, and thus improved chronic fixation.

Finally, the preferred sleeve requires less material (40 denier yarn as compared to 80 denier yarn).

As the preferred sleeve is elongated, the yarn cross-section aspect ratio is reduced from 3.66 before elongation to 1.15 at complete elongation. This illustrates the extent to which the yarn cross-section undergoes changes in its profile to accommodate the axial elongation. By contrast, the less preferred circular cross-sectional yarn retains its aspect ratio of unity during elongation. The fabric thickness of the sleeve employing the twisted yarn remains essentially the same during axial elongation, although slightly decreasing near the end. The fabric thickness of the preferred sleeve also remains constant through most of the axial elongation, but increases substantially near the end because of the reduced yarn diameter resulting from increased fiber packing.

In the twisted-yarn sleeve, yarn coverage increases with axial elongation, while fabric porosity decreases. In the preferred sleeve, yarn coverage decreases significantly near the end of axial elongation, while fabric porosity remains generally constant. In both sleeves, axial elongation increases the packing factor.

The above trends and comparative values notwithstanding, it is to be understood that fabric sleeve performance is governed by yarn coverage, fabric porosity, yarn aspect ratio and packing factor at or near the nominal state, i.e. with little or no axial elongation.

Further in accordance with this invention, parameters relating to the yarns and fabrics can be tailored to achieve desired physical properties.

In general, thinner walls for the fabric sleeve are preferred, so long as the fabric sleeve meets water permeability, longitudinal strength and radial burst strength requirements. Yarn cross-sectional aspect ratios in the preferred range of 2-12 permit substantial thinning of the fabric sleeve without sacrificing longitudinal or radial strength.

The filament or fiber packing factor should be optimized to balance the desire for tissue ingrowth with the need for graft permeability. The filament packing in textured yarns is optimal at packing factors of 0.50-0.55 in the unconstrained state. Flat yarns are characterized by higher optimum densities, with favored packing factors from about 0.60 to about 0.65 in the unconstrained state.

The selection of yarn cross-sectional aspect ratios also require optimizing. Higher aspect ratios lead to improved axial elongation properties. However, they also reduce the degree of yarn (filament) interlocking, thus tending to reduce fabric stability. The preferred aspect ratio range of 2-12 has been found to satisfy these competing needs, with the lower end of the range favoring stability while the higher end favors enhanced elongation.

The yarn coverage ratio preferably is as high as possible to achieve minimum yarn interstices, while fabric porosity is kept low to meet water permeability requirements.

The number of yarn ends should be fixed after determining several other factors, including braid angle, sleeve diameter, fabric thickness, yarn linear density, packing factor, and aspect ratio.

Table 3 illustrates several examples of fabric sleeves for use in stent grafts. In all cases, the textile strands have a packing factor of 0.54, and are braided at a braid angle of 110 degrees.

Table 3

| Inner Diameter | Number of Yarn Ends | Yarn Linear Density | Fabric ② Thickness | Yarn Coverage | Fabric Porosity | Yarn Aspect Ratio |
|---|---|---|---|---|---|---|
| 6 mm | 72 | 70 | 0.0031" | 98% | 55% | 6.53 |
| 6 mm | 96 | 50 | 0.0032" | 97% | 58% | 4.62 |
| 6 mm | 120 | 40 | 0.0034" | 94% | 62% | 3.15 |
| 6 mm | 144 | 30 | 0.0032" | 93% | 64% | 2.69 |
| 12 mm | 192 | 50 | 0.0032" | 97% | 58% | 4.62 |
| 24 mm | 352 | 60 | 0.0035" | 97% | 58% | 4.56 |
| 40 mm | 512 | 70 | 0.0034" | 98% | 56% | 5.45 |

② 0.0031" = 0.078 mm
0.0032" = 0.08 mm
0.0034" = 0.085 mm
0.0035" = 0.087 mm

Thus in accordance with the present invention, a stent graft is formed with distinct layers to provide the structural advantages of stents with the capacity of grafts for shunting blood and other body fluids. The stent graft is adjustable in size by simultaneous radial reduction and axial elongation, or simultaneous radial enlargement and axial shortening. The separate layers are configured to behave according to substantially the same relationship of radial change with respect to axial change, so that the graft behaves as a unitary structure during radial enlargements and reductions. The layered construction involves independent formation of latticework and microporous sleeves followed by their bonding, a process that reduces cost and promotes a customizing of stent grafts through selective positioning of sleeves to leave areas of the latticework exposed.

**Claims**

1. A process for making a stent graft, comprising:

   forming a plurality of the structural strands (36) into a tubular open latticework (34, 106, 116, 130) having a nominal state and in which the structural strands intersect one another to define at each intersection (70) a first angle, said latticework being adjustable between the nominal state and a radially-reduced, axially-elongated state according to a first relationship of radial reduction versus axial elongation, wherein radial compression of the latticework reduces the first angle;

   forming a plurality of compliant textile strands (42) into a tubular sleeve (40, 108) having a nominal state and in which the textile strands intersect one another to define at each intersection a second angle, wherein radial compression of the sleeve reduces the second angle; and

   disposing a selected one of the latticework and sleeve within and in an axial alignment with the other of the latticework and sleeve, so that said other surrounds the selected one, and joining the latticework and sleeve to one another with an adhesive layer (44) along an interface between the latticework and sleeve, to form a composite stent graft (18, 90, 92, 104, 112, 124, 128);

**characterized in that** after said joining the latticework and sleeve, the structural strands and the textile strands, at least over a medial region of the composite stent graft, remain free to move relative to one another at their respective intersections whereby radial compression of the composite stent graft reduces the first angle and the second angle; and

further **characterized in that** the tubular sleeve is adjustable between its nominal state and a radially-reduced axially-elongated state according to a second relationship of radial reduction versus axial elongation substantially equivalent to said first relationship, to minimize any stress or stiffness occasioned by differences in axial elongation rates as the stent graft is radially compressed.

2. The process of claim 1 wherein:

said forming of the structural strands (36) includes arranging the structural strands as first and second sets of helices wound in opposite directions about a common longitudinal axis (38), thereby forming rhombic interstices between adjacent structural strands.

3. The process of claim 2 wherein:

said forming of the structural strands (36) further includes interbraiding the structural strands.

4. The process of claim 2 wherein:

said forming of the textile strands (42) includes arranging the textile strands as third and fourth sets of helices wound in opposite directions about the common longitudinal axis (38).

5. The process of claim 4 wherein:

the second angle is larger than the first angle, and the sleeve surrounds the latticework.

6. The process of claim 4 wherein:

the second angle is smaller than the first angle, and the sleeve is surrounded by the latticework.

7. The process of any one of claims 2 to 6 wherein:

said structural strands (36) are thermally formable, and the forming of the structural strands includes winding the structural strands around a selectively sized first mandrel (58), then heating the structural strands while so wound to a first heat forming temperature sufficient to thermally impart the nominal state and a corresponding nominal size and shape to the latticework (34, 106, 116, 130).

8. The process of claim 7 wherein:

said textile strands (42) are thermally formable, and the forming of the textile strands includes winding the textile strands around a selectively sized second mandrel (62), then heating the textile strands while so wound to a second heat forming temperature sufficient to thermally impart the nominal state and a corresponding nominal size and shape to the sleeve (40, 108).

9. The process of any preceding claim wherein:

the first mandrel (58) has a diameter less than that of the second mandrel (62).

10. The process of any preceding claim wherein:

the second angle is within one degree of the first angle.

11. The process of claim 8 wherein:

the first mandrel (58) and the second mandrel (62) have substantially the same diameter, and the first angle is larger than the second angle.

**12.** The process of any preceding claim further including:

prior to said disposing, applying an adhesive to at least one of the latticework (34, 106, 116, 130) and sleeve (40, 108) over at least a portion of its axial length, wherein said attaching includes maintaining the latticework and the sleeve in said engagement for a time sufficient for the adhesive to bond the latticework and the sleeve to form the composite stent graft (18, 90, 92, 104, 112, 124, 128).

**13.** The process of claim 12 wherein:

said applying includes dissolving the adhesive in a liquid solvent, spraying the combined solvent and adhesive onto the at least one of the latticework (34, 106, 116, 130) and sleeve (40, 108), then evaporating the solvent to leave a residue consisting essentially of the adhesive.

**14.** The process of claim 12 or claim 13 wherein:

said applying further includes providing the adhesive in the form of a siloxane polymer and dissolving the siloxane polymer in an organic solvent at about six percent siloxane polymer by weight.

**15.** The process of any one of claims 12 to 14 wherein:

said adhesive is heat curable, and said attaching includes maintaining the composite stent graft (18, 90, 92, 104, 112, 124, 128) at a curing temperature of about 125-200 degrees C.

**16.** The process of any one of claims 12 to 14 wherein:

said adhesive is curable, and applied uncured to the at least one of the latticework (34, 106, 116, 130) and sleeve (40, 108), and said attaching includes curing the adhesive.

**17.** The process of any one of claims 12 to 16:

said applying includes electrostatically depositing an adhesive onto the at least one of the latticework (34, 106, 116, 130) and sleeve (40, 108).

**18.** The process of claim 16 further including:

after said curing, plastically deforming the latticework (34, 106, 116, 130) to radially reduce the latticework and the sleeve (40, 108) to their respective radially-reduced axially-elongated states.

**19.** The process of any preceding claim wherein:

said disposing includes adjusting the selected one of the latticework (34, 106, 116, 130) and sleeve to reduce its radius below that in its nominal state, axially aligning the selected one with the other while the other remains in its nominal state, then radially expanding the selected one toward said engagement with the other.

**20.** A body insertable stent graft including:

a radially expandable stent (34, 106, 116, 130) comprising a plurality of structural strands (36) intersecting one another to define at each intersection (70) a first angle, said stent being adjustable between a nominal state and a radially-reduced axially-elongated state according to a first relationship of radial reduction versus axial elongation;
a tubular sleeve (40, 108) formed of a plurality of textile strands (42) intersecting one another to define at each intersection a second angle; and
an adhesive layer (44) along an interface between the stent and the sleeve joining the stent and sleeve in an axial alignment with a selected one of the stent and sleeve surrounding the other to form a composite stent graft (18, 90, 92, 104, 112, 124, 128);

**characterized in that** the structural strands (36) and the textile strands (42), at least over a medial region of the composite stent graft, are free to move relative to one another at their respective intersections whereby radial compression of the composite stent graft reduces the first and second angles; and

further **characterized in that** the tubular sleeve (40, 108) is adjustable between a nominal state and a radially-reduced axially-elongated state according to a second relationship of radial reduction versus axial elongation substantially equivalent to said first relationship, to minimize any stress or stiffness occasioned by differences in axial elongation rates as the stent graft is radially compressed.

**21.** The stent graft of claim 20 wherein:

the structural strands (36) are wound in first and second sets of helices in opposite directions about a longitudinal axis (38) of the stent graft (18, 90, 92, 104, 112, 124, 128) to form the first angle when the stent is in its nominal state; and

the textile strands (42) are wound in third and fourth sets of helices in opposite directions about the longitudinal axis to form the second angle when the sleeve (40, 108) is in its nominal state; and

the second angle is within about five degrees of the first angle.

**22.** The stent graft of claim 20 or claim 21 wherein:

the structural strands (36) are resilient, whereby the stent tends to assume its nominal state when relaxed.

**23.** The stent graft of any one of claims 20 to 22 wherein:

the sleeve (40, 108) surrounds the stent (34, 106, 116, 130).

**24.** The stent graft of any one of claims 20 to 23 wherein:

the structural strands (36) are interbraided.

**25.** The stent graft of any one of claims 20 to 24 wherein:

the textile strands (42) are interbraided.

**26.** The stent graft of any one of claims 20 to 25 wherein:

the structural strands are monofilaments constructed of at least one of the following materials: stainless steel, an alloy including cobalt, an alloy including titanium, PET, polypropylene, PEEK, HDPE, polysulfone, PTFE, FEP, and polyurethane.

**27.** The stent graft of any one of claims 20 to 25 wherein:

the textile strands are multifilament yarns formed of at least one of the following materials: PET, polypropylene, high molecular weight polyethylene, polyurethane, HDPE, polyethylene, silicone, PTFE, and polyolefin.

**28.** The stent graft of any one of claims 20 to 27 wherein:

the adhesive layer (44) consists essentially of a siloxane polymer.

**29.** The stent graft of claim 28 wherein:

the siloxane polymer occupies at least proximal and distal end portions of the interface region.

**30.** The stent graft of claim 28 wherein:

said siloxane polymer occupies substantially all of said interface region.

**31.** The stent graft of any one of claims 21 to 30 wherein:

the second angle is between 100.91 percent and 105.45 percent of the first angle, and the sleeve surrounds the stent.

**32.** The stent graft of any one of claims 21 to 30 wherein:

the second angle is between 97.72 percent and 102.27 percent of the first braid angle, and the sleeve is surrounded by the stent.

**33.** The stent graft of any one of claims 21 to 32 wherein:

each textile strand (42) consists essentially of a multifilament yarn (142) in which multiple filaments (144) thereof are arranged to define a yarn cross-section having an aspect ratio of at least 2.

**34.** The stent graft of claim 33 wherein:

the textile strands (42) are oriented with their minimum cross-sectional dimensions aligned substantially radially of the sleeve (40, 108).

**35.** The stent graft of any one of claims 20 to 34 wherein:

each of the textile strands (42) consists essentially of a multifilament yarn (142) in which the filaments (144) have surface twist angles of at most 15 degrees.

FIG-1

FIG-2

# FIG-3

# FIG-4

# FIG-5

# FIG-6

# FIG-7

# FIG-8

FIG-9

FIG-10

# FIG-11

90

86

88

84

# FIG-12

92

98

100

94

96

102

FIG-13

FIG-14

FIG-15

FIG-16

FIG-17

EP 1 493 402 A2

FIG-18

FIG-19

FIG-20